Europäisches Patentamt

European Patent Office

Office européen des brevets

(19)

(11)     EP 0 920 877 B1

(12)         **FASCICULE DE BREVET EUROPEEN**

(45) Date de publication et mention
de la délivrance du brevet:
**07.04.2004   Bulletin 2004/15**

(51) Int Cl.⁷: $A61M\ 1/16$

(21) Numéro de dépôt: **98420228.3**

(22) Date de dépôt: **08.12.1998**

(54) **Procédé de détermination d'un paramètre significatif du progrès d'un traitement extracorporel de sang**

Verfahren zur Bestimmung eines signifikanten Parameters für den Fortschritt einer extrakorporalen Blutbehandlung

Procedure for determining a parameter indicating the progress of an extracorporeal blood treatment

(84) Etats contractants désignés:
**AT BE CH DE ES FR GB GR IT LI NL SE**

(30) Priorité: **09.12.1997   FR 9715818**

(43) Date de publication de la demande:
**09.06.1999   Bulletin 1999/23**

(73) Titulaires:
• **HOSPAL INDUSTRIE**
**F-69883 Meyzieu Cédex (FR)**
• **Gambro Lundia AB**
**220 10 Lund (SE)**

(72) Inventeurs:
• **Goux, Nicolas**
**69290 Craponne (FR)**
• **Sternby, Jan**
**222 52 Lund (SE)**

(74) Mandataire: **Sutto, Luca et al**
**Gambro Intellectual Property Department**
**Hospal International Marketing Management**
**61, avenue Tony Garnier**
**69007 Lyon (FR)**

(56) Documents cités:
**EP-A- 0 428 927          EP-A- 0 547 025**
**EP-A- 0 658 352          WO-A-95/32010**

**Description**

L'invention concerne un procédé de détermination d'un paramètre significatif du progrès d'un traitement extracorporel de sang, en particulier d'un traitement d'épuration ayant pour but de pallier l'insuffisance rénale, comme l'hémodialyse ou l'hémodiafiltration.

Pour mémoire, l'hémodialyse consiste à faire circuler, de part et d'autre de la membrane semi-perméable d'un échangeur, le sang d'un patient et un liquide de traitement sensiblement isotonique au sang, de façon que, lors du transfert diffusif qui s'établit au travers de la membrane pour les substances ayant des concentrations différentes de part et d'autre de la membrane, les impuretés du sang (urée, créatinine, etc.) migrent du sang vers le liquide de traitement. La concentration électrolytique du liquide de traitement est aussi généralement choisie pour corriger la concentration électrolytique du sang du patient.

Dans le traitement par hémodiafiltration, au transfert diffusif obtenu par dialyse s'ajoute un transfert convectif par ultrafiltration résultant d'une différence de pression positive créée entre le coté sang et le coté liquide de traitement de la membrane.

Il est du plus grand intérêt de pouvoir déterminer, tout au long d'une séance de traitement, un ou plusieurs paramètres significatifs du progrès du traitement afin de pouvoir, le cas échéant, modifier les conditions du traitement telles qu'elles ont été fixées initialement en vue d'un objectif thérapeutique déterminé.

Les paramètres dont la connaissance permet de suivre le progrès du traitement, c'est-à-dire aussi d'apprécier l'adéquation des conditions de traitement fixées initialement à l'objectif thérapeutique, sont, en particulier, la concentration du sang en un soluté donné (sodium par exemple), ou la dialysance réelle ou la clairance réelle de l'échangeur pour tel ou tel soluté (la dialysance et la clairance représentant le rendement épuratif de l'échangeur) ou la dose de dialyse administrée après un temps t de traitement, laquelle, d'après les travaux de Sargent et Gotch, peut être assimilée au rapport sans dimension Kt/V, où K est la clairance réelle pour l'urée, t le temps de traitement écoulé, et V le volume de distribution de l'urée, c'est-à-dire le volume d'eau total du patient (Gotch FA, Sargent SA. A mechanistic analysis of the National Cooperative Dialysis Study (NCDS). Kidney int 1985 ; 28 : 526-34).

Ces paramètres posent tous le même problème pour leur détermination, qui est de nécessiter la connaissance précise d'une caractéristique physique ou chimique du sang, alors que cette caractéristique ne peut pas, dans la pratique, être obtenue par mesure directe sur un échantillon pour des raisons thérapeutiques, prophylactiques et pécuniaires : en premier lieu, il est exclu de prélever sur un patient, souvent anémié, les échantillons multiples qui seraient nécessaires pour suivre l'efficacité du traitement au cours de son déroulement ; en outre, compte tenu des risques liés à la manipulation d'échantillons de sang éventuellement contaminé, la tendance générale est à éviter de telles manipulations ; enfin, l'analyse d'un échantillon de sang en laboratoire est à la fois coûteuse et relativement longue, ce qui est incompatible avec l'objectif recherché.

Le document EP 0 658 352 décrit un procédé pour la détermination in vivo des paramètres de l'hémodialyse ne nécessitant pas d'effectuer des mesures sur le sang. Ce procédé, dont la mise en oeuvre requiert des moyens pour régler la concentration ionique du liquide de traitement et des moyens pour mesurer la concentration en sodium du liquide de traitement ou sa conductivité, comprend les étapes de:

- faire circuler successivement dans l'échangeur au moins un premier (d1) et un second (d2) liquides de traitement ayant une caractéristique (Cd) liée à au moins un des paramètres significatifs du traitement (Cb, D, K, Kt/V), la valeur de la caractéristique dans le premier liquide (d1) en amont de l'échangeur étant différente de la valeur de la caractéristique (Cd) dans le second liquide (d2) en amont de l'échangeur,
- mesurer dans chacun des premier (d1 ) et second (d2) liquides de traitement deux valeurs (Cd1in, Cd1out ; Cd2in, Cd2out) de la caractéristique (Cd), respectivement en amont et en aval de l'échangeur ;
- mettre en circulation un troisième (d3) liquide de traitement dans l'échangeur alors que la caractéristique (Cd) du second liquide (d2) n'a pas atteint une valeur stable en aval de l'échangeur, la valeur de la caractéristique (Cd) dans le troisième liquide (d3) en amont de l'échangeur étant différente de la valeur de la caractéristique (Cd) dans le second liquide (d2) en amont de l'échangeur,
- mesurer deux valeurs (Cd3in, Cd3out) de la caractéristique (Cd) dans le troisième liquide (d3) respectivement en amont et en aval de l'échangeur, et
- calculer au moins une valeur d'au moins un paramètre significatif du progrès du traitement (Cb, D, K, Kt/V) à partir des valeurs mesurées de la caractéristique (Cd) dans le premier (d1) le second (d2) et le troisième (d3) liquides de traitement.

Un but de l'invention est de concevoir un procédé de détermination d'un paramètre significatif du progrès d'un traitement extracorporel de sang qui soit au moins aussi fiable que le procédé mentionné ci-dessus, c'est-à-dire grâce auquel les paramètres représentatifs du progrès du traitement puissent être déterminés fréquemment, de façon exacte, et sans que le patient doive être durablement soumis à des conditions de traitement différentes des conditions prescrites.

Pour atteindre ce but, on prévoit, selon l'invention, un procédé de détermination d'un paramètre (D, K, Kt/v, Cbin) significatif de l'efficacité d'un traitement extracorporel du sang consistant à faire circuler de part et d'autre de la membrane semi-perméable d'un échangeur à membrane le sang d'un patient et un liquide de traitement, caractérisé en ce qu'il comporte les étapes de :

- faire circuler dans l'échangeur un liquide de traitement ayant une caractéristique (Cd) ayant une valeur nominale (Cd0in) sensiblement constante en amont de l'échangeur;
- faire varier la valeur de la caractéristique (Cd) en amont de l'échangeur pendant une durée tc - ta, à la fin de laquelle la caractéristique (Cd) est rétablie à sa valeur nominale (Cd0in) en amont de l'échangeur;
- mesurer et mettre en mémoire une pluralité de valeurs prises par la caractéristique (Cd) du liquide de traitement en aval de l'échangeur en réponse à la variation de la valeur de cette caractéristique (Cd) provoquée en amont de l'échangeur;

caractérisé en ce qu'il comporte les étapes de:

- déterminer la superficie (Sout) d'une zone de perturbation aval délimitée par:

  • une ligne de base passant par deux valeurs prises par la caractéristique (Cd) à deux instants encadrant la perturbation, auxquels la caractéristique (Cd) n'est pas, respectivement plus, influencée par la perturbation, et
  • une courbe représentative de l'évolution par rapport au temps de la caractéristique (Cd), établie à partir des valeurs de la caractéristique (Cd) mesurées en aval de l'échangeur entre deux instants t1 et t3 encadrant la perturbation et auxquels la caractéristique (Cd) n'est pas, respectivement plus, influencée par la perturbation ; et

- calculer le paramètre (D, K, Kt/v, Cbin) significatif de l'efficacité d'un traitement à partir de la superficie (Sout) de la zone de perturbation aval et de la superficie (Sin) d'une zone de perturbation amont délimitée par:

  • une ligne de base constituée par la valeur nominale (Cd0in) de la caractéristique (Cd), et
  • une courbe représentative de l'évolution par rapport au temps, pendant la durée tc - ta, de la caractéristique (Cd) en amont de l'échangeur.

Ce procédé présente l'intérêt de permettre une détermination précise des paramètres significatifs du progrès du traitement à partir de mesures effectuées à intervalles de temps rapprochés. De la sorte, le patient n'est exposé que pendant un temps très court à un liquide de traitement différent du liquide de traitement prescrit (par exemple trop riche ou trop pauvre en sodium) et le procédé peut être mis en oeuvre aussi souvent que nécessaire pour un suivi approprié de la séance de traitement. Par ailleurs, dans la mesure où les grandeurs qui interviennent dans les calculs effectués selon ce procédé sont des surfaces et non pas des valeurs isolées, ce procédé est peu sensible aux incidents de tous ordres qui peuvent se produire au moment de la mesure d'une valeur isolée et qui peuvent fausser les calculs ultérieurs faisant intervenir une valeur erratique.

Selon une caractéristique de l'invention, l'étape de faire varier la valeur de la caractéristique (Cd) du liquide de traitement en amont de l'échangeur comprend les étapes de :

- régler, pendant un premier intervalle de temps déterminé, la caractéristique à une première valeur de consigne déterminée supérieure (respectivement inférieure) à la valeur nominale (Cd0in), et
- régler, pendant un deuxième intervalle de temps, la caractéristique à une deuxième valeur de consigne inférieure (respectivement supérieure) à la valeur nominale (Cd0in).

De préférence, la durée du deuxième intervalle de temps ainsi que la valeur absolue de la deuxième valeur de consigne sont choisies de façon que la caractéristique (Cd) reprenne sa valeur initiale (Cd0out) en aval de l'échangeur en un temps minimal.

Selon une autre caractéristique de l'invention, le procédé comprend en outre, préalablement à l'étape de faire varier la valeur de la caractéristique (Cd), les étape de:

- donner aux paramètres de la variation une valeur définie, de façon que la superficie de la zone de perturbation amont Sin soit constante quand les paramètres prédéterminés de la variation sont respectés;
- déterminer et mettre en mémoire la superficie de la zone de perturbation amont Sin pour une variation ayant les paramètres fixés.

Lorsque la caractéristique soumise à variation est la concentration dans le liquide de traitement d'une substance présente dans le liquide de traitement et dans le sang, il est possible, selon l'invention, de calculer la dialysance D de l'échangeur pour cette substance et/ou la concentration Cbin de la substance dans le sang du patient en amont de l'échangeur au moyen des formules suivantes:

$$D = (Qd + Quf) \times (1 - Sout/Sin)$$

$$Cbin = \frac{(Cd0in \times Sout/Sin + Cd0out)}{(1 - Sout/Sin)}$$

D'autres caractéristiques et avantages de l'invention apparaîtront à la lecture de la description qui suit. On se reportera aux dessins sur lesquels :

La figure 1 est une représentation schématique partielle d'un système d'hémodialyse/hémodiafiltration adaptée à la mise en oeuvre du procédé selon l'invention;
La figure 2 est un diagramme représentant l'évolution de la conductivité du liquide de traitement en fonction du temps lors de la mise en oeuvre du procédé selon l'invention;
La figure 3 est un diagramme représentant un exemple de consignes de conductivité pour un liquide de dialyse appropriées à la mise en oeuvre de l'invention; et
La figure 4 est un diagramme représentant l'évolution de la conductivité d'un liquide de dialyse en amont et en aval d'un dialyseur en réponse aux consignes de conductivité représentées sur la figure 3.

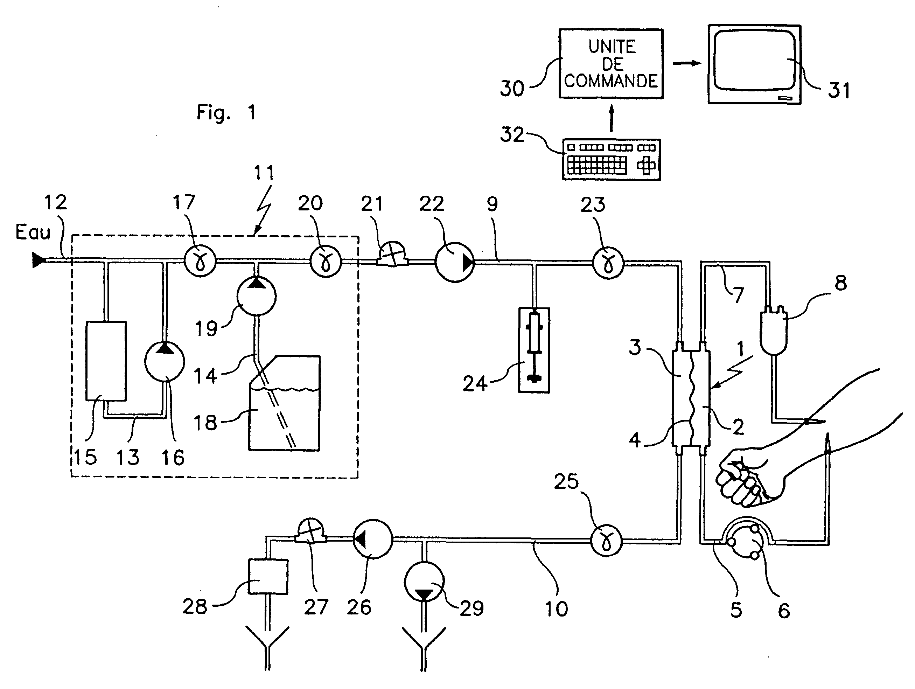

Le système d'hémodialyse représenté sur la figure 1 comprend un hémodialyseur 1 ayant deux compartiments 2, 3 séparés par une membrane semi-perméable 4. Un premier compartiment 2 a une entrée connectée à une canalisation 5 de prélèvement de sang sur laquelle est disposée une pompe de circulation 6, et une sortie connectée à une canalisation 7 de restitution de sang sur laquelle est interposé un piège à bulles 8.

Le second compartiment 3 de l'hémodialyseur 1 a une entrée connectée à une canalisation 9 d'alimentation en liquide de dialyse frais et une sortie connectée à une canalisation 10 d'évacuation de liquide usé (liquide de dialyse et ultrafiltrat).

La canalisation d'alimentation 9 relie l'hémodialyseur 1 à un dispositif de préparation de liquide de dialyse 11 comprenant une canalisation principale 12 dont l'extrémité amont est prévue pour être raccordée à une source d'eau courante. A cette canalisation principale 12 sont connectées une première et une deuxième canalisation secondaires 13, 14. La première canalisation secondaire 13, qui est rebouclée sur la canalisation principale 12, est munie de moyens de raccord pour le montage d'une cartouche 15 contenant du bicarbonate de sodium sous forme de granulés. Elle est en outre équipée d'une pompe 16 de dosage du bicarbonate dans le liquide de dialyse, disposée en aval de la cartouche 15. La pompe 16 est pilotée en fonction de la comparaison entre 1) une première valeur de consigne de conductivité pour la solution se formant à la jonction de la canalisation principale 12 et de la canalisation secondaire 13 et 2) la valeur de la conductivité de ce mélange mesurée au moyen d'une première sonde de conductivité 17 disposée sur la canalisation principale 12 immédiatement en aval de la jonction entre la canalisation principale 12 et la première canalisation secondaire 13.

L'extrémité libre de la deuxième canalisation secondaire 14 est destinée à être immergée dans un conteneur 1 8 pour une solution saline concentrée contenant du chlorure de sodium, de calcium, de magnésium et de potassium, ainsi que de l'acide acétique. La seconde canalisation 14 est équipée d'une pompe 19 de dosage du sodium dans le liquide de dialyse qui est pilotée en fonction de la comparaison entre 1) une seconde valeur de consigne de conductivité pour la solution se formant à la jonction de la canalisation principale 12 et de la seconde canalisation secondaire 14 et 2) la valeur de la conductivité de cette solution mesurée au moyen d'une deuxième sonde de conductivité 20 disposée sur la canalisation principale 12 immédiatement en aval de la jonction entre la canalisation principale 12 et la canalisation secondaire 14.

La canalisation d'alimentation 9 forme le prolongement de la canalisation principale 12 du dispositif 11 de préparation de liquide de dialyse. Sur cette canalisation d'alimentation sont disposés, dans le sens de circulation du liquide, un premier débitmètre 21, une première pompe de circulation 22 et une troisième sonde de conductivité 23. Des moyens 24 d'injection permettant d'injecter un volume précis de liquide, à un débit précis, tels qu'une seringue montée dans un pousse seringue, sont connectés à la canalisation d'alimentation 9 entre la première pompe de circulation 21 et la troisième sonde de conductivité 2 .

L'extrémité aval de la canalisation 10 d'évacuation de liquide usé est prévue pour être reliée à l'égout. Sur cette canalisation sont disposés, dans le sens de circulation du liquide, une troisième sonde de conductivité 25, une deuxième pompe de circulation 26, un deuxième débitmètre 27 et un dispositif de mesure de l'urée 28. Une pompe d'extraction 29 est reliée à la canalisation d'évacuation 10, en amont de la deuxième pompe de circulation 26.

Le système d'hémodialyse représenté sur la figure 1 comprend également une unité de calcul et de commande 30. Cette unité est reliée à un écran 31 et à un clavier 32 par lequel l'utilisateur lui communique diverses valeurs de consigne : consignes de débit (débit de sang Qb, débit de liquide de dialyse Qd), consignes de conductivité utilisées pour la préparation du liquide de dialyse, consigne de durée de traitement T, consigne de perte de poids WL. Par ailleurs, l'unité de calcul et de commande 30 reçoit des information émises par les organes de mesure du système, tels que les débitmètres 21, 27, les sondes de conductivité 17, 20, 23, 25, le dispositif de mesure de l'urée 28. Elle pilote, en fonction des instructions reçues et de modes d'opération et d'algorithmes programmés les organes moteurs du système, tels que les pompes 6, 16, 19, 22, 26, 29.

Le système d'hémodialyse qui vient d'être décrit fonctionne de la façon suivante.

Après que le circuit extracorporel de sang a été rincé et rempli de solution saline stérile, il est connecté au patient et, la pompe à sang 6 est mise en service à un débit prédéterminé Qb, 200 ml/min par exemple.

Simultanément, les pompes 16 et 19 du dispositif de préparation de liquide de dialyse 11, -les pompes de circulation de liquide de dialyse 22, 26 et la pompe d'extraction 29 sont mises en service. Le débit des pompes de dosage 16, 19 est réglé au moyen des sondes de conductivité 17, 20 pour que le liquide de dialyse ait la concentration en bicarbonate et la concentration en sodium souhaitées. Le débit Qd de la pompe de circulation 22 disposée sur la canalisation d'alimentation 9 est réglé à une valeur fixe (500 ml/min, par exemple), tandis que le débit de la pompe de circulation 26 disposée sur la canalisation d'évacuation 10 est ajusté en permanence de façon que le débit mesuré par le second débitmètre 27 soit égal au débit mesuré par le premier débitmètre 21. Le débit de la pompe d'extraction 29 est réglé pour être égal au débit de perte de poids (calculé à partir du poids WL qu'il est prescrit de faire perdre au patient et de la durée T de la séance de traitement) augmenté éventuellement du débit d'un liquide perfusé au patient.

Conformément à l'invention, l'efficacité du traitement administré au patient au moyen du système qui vient d'être décrit est déterminé soit à tout moment au choix du médecin, soit à intervalles de temps programmés, au moyen du procédé suivant.

Défini de façon générale, le procédé de détermination d'un paramètre significatif de l'efficacité du traitement selon l'invention consiste 1) à créer une perturbation dans le régime d'échanges établi dans l'hémodialyseur 1 en modifiant pendant un court espace de temps (deux à trois minutes par exemple, alors qu'une séance d'hémodialyse dure environ quatre heures) une caractéristique stable du liquide de dialyse, 2) à déterminer une grandeur significative de la variation imposée à la caractéristique du liquide de dialyse en amont de l'hémodialyseur, 3) à déterminer une grandeur significative de la variation de la caractéristique telle qu'elle apparaît en aval de l'hémodialyseur 1 déformée par les échanges ayant lieu, pour cette caractéristique, dans l'hémodialyseur 1 et 4) à calculer un paramètre significatif de l'efficacité du traitement administré à partir de la grandeur significative de la variation de la caractéristique en amont de l'hémodialyseur et de la grandeur significative de la variation de la caractéristique en aval de l'hémodialyseur.

De façon détaillée, les étapes du procédé peuvent être réalisées de la façon suivante.

1 - Création d'une perturbation dans le régime d'échanges établi dans l'hémodialyseur 1.

La concentration d'une substance réglée, pendant le fonctionnement ordinaire du système, à une valeur stable Cd0in dans le liquide de dialyse, est modifiée.

Cette substance peut être le sodium auquel cas la variation de la concentration du sodium dans le liquide de dialyse peut être obtenue soit en faisant varier le débit de la pompe de dosage du sodium 19 selon une séquence programmée, soit en injectant dans le liquide de dialyse frais, au moyen du pousse seringue 24, un volume déterminé d'une solution de chlorure de sodium ayant une concentration supérieure ou inférieure à la concentration de sodium Cd0in prescrite pour le liquide de dialyse.

La variation de la concentration en sodium du liquide de dialyse, c'est-à-dire aussi de sa conductivité, peut consister en une augmentation momentanée de la concentration, en une diminution momentanée de la concentration, ou en une séquence augmentation/diminution momentanée de la concentration ou l'inverse (une telle séquence ne peut être mise en oeuvre qu'au moyen de la pompe 19 de dosage de sodium, ou il faut disposer de deux pousse-seringues contenant des solutions de chlorure de sodium dont la concentration est respectivement supérieure et inférieure à la concentration du liquide de dialyse Cd0in).

Bien que cette étape du procédé puisse être mise en oeuvre par l'injection, dans la canalisation d'alimentation 9, d'un volume indéterminé, à un débit plus ou moins variable, d'une solution de chlorure de sodium dont la concentration en sodium n'est pas précisément connue, il est avantageux de programmer les paramètres de la variation dans l'unité de commande 30. Dans le cas où la variation est provoquée par la pompe 19 de dosage de sodium, la variation sera alors définie par un débit spécifique de la pompe (supérieur, ou inférieur, ou supérieur puis inférieur, ou l'inverse, au débit nominal de la pompe) et l'intervalle (ou les intervalles) de temps pendant le(s)quel(s) la pompe 19 sera pilotée à ce débit (ou à ces débits) spécifique(s). Dans le cas où la variation est provoquée par le pousse seringue 24, une variation calibrée sera définie par la concentration en sodium de cette solution, par le volume de solution injecté et par le débit d'injection.

Selon une variante de mise en oeuvre du procédé selon l'invention, au lieu de faire varier, dans le liquide de dialyse, la concentration d'une substance présente dans le sang et dans le liquide de dialyse, on fait varier la concentration d'une substance présente dans le sang et absente du liquide de dialyse, telle que l'urée. On utilise alors le pousse seringue 24 pour injecter dans le liquide de dialyse une solution isotonique au sang contenant de l'urée.

Comme cela apparaîtra clairement plus tard, le procédé selon l'invention livre des informations d'autant plus fiables qu'il se déroule pendant un stade de fonctionnement très stable du système d'hémodialyse, où tous les paramètres de fonctionnement du système, à l'exception de la caractéristique du liquide de dialyse que l'on a choisi de faire varier, restent sensiblement constants (débits du sang et du liquide de dialyse, en particulier). Il est donc intéressant de faire en sorte que la mise en oeuvre du procédé soit brève, c'est-à-dire aussi de conformer la variation induite dans le liquide de dialyse frais de telle façon que la conductivité du liquide de dialyse en aval de l'hémodialyseur 1 tende aussi rapidement que possible la valeur initiale CdOout qu'elle avait avant la perturbation.

A cette fin, il est avantageux de programmer une variation de la conductivité qui comprenne un premier échelon d'intensité et de durée déterminées (par exemple +1 mS/cm par rapport à la conductivité nominale Cd0in, pendant 60 secondes) suivi d'un échelon de sens opposé d'intensité déterminée (par exemple -1 mS/cm par rapport à la conductivité nominale Cd0in). La durée du second échelon peut être fixée à une valeur déterminée (par exemple, 30 secondes) qui aura été choisie empiriquement à partir d'essais pratiqués sur divers types d'hémodialyseur, pour des débits de liquide de dialyse et de sang situés dans les fourchettes de débits usuellement utilisés en hémodialyse. La durée du contre-échelon peut aussi être ajustée par l'unité de commande 30 en fonction de la conductivité du liquide usé mesurée par la sonde de conductivité 25 disposée sur la canalisation d'évacuation 10. Dans une variante plus sophistiquée du procédé, l'unité de commande 30 ajuste, en fonction de l'évolution de la conductivité en aval de l'hémodialyseur 1 résultant de l'échelon, à la fois l'intensité et la durée du contre échelon de façon que la conductivité mesurée par la sonde 25 tende le plus rapidement possible après la perturbation vers la valeur de conductivité stable initiale Cd0out.

2 - Détermination d'une grandeur significative de la variation imposée à la caractéristique choisie du liquide de dialyse.

Dans la suite, à titre d'exemple, on considéra que cette caractéristique est la concentration en sodium du liquide de dialyse, c'est-à-dire aussi sa conductivité. Conformément à l'invention, la grandeur choisie est la superficie Sin d'une zone appelée "zone de perturbation amont", qui est déterminée comme suit.

Le système d'hémodialyse fonctionnant à un régime d'échange stable pour ce qui est de la concentration du sodium, la conductivité Cd0in du liquide de dialyse frais, telle que mesurée par la sonde 23, étant égale à la conductivité de la prescription (ou conductivité nominale) et la conductivité Cd0out du liquide de dialyse usé, telle que mesurée par la sonde 25, étant sensiblement constante, l'unité de commande 30 provoque, à un moment déterminé, une variation de la conductivité du liquide de dialyse en pilotant la pompe 19 ou le pousse seringue 24 de la façon qui a été décrite plus haut. La sonde de conductivité 23 mesure en continu la variation de la conductivité du liquide de dialyse frais, tandis que l'unité de commande 30 échantillonne et met en mémoire, à intervalles de temps $\Delta t$ déterminés, une pluralité de valeurs de conductivité Cdiin aussi longtemps que la conductivité n'a pas repris sa valeur nominale Cd0in.

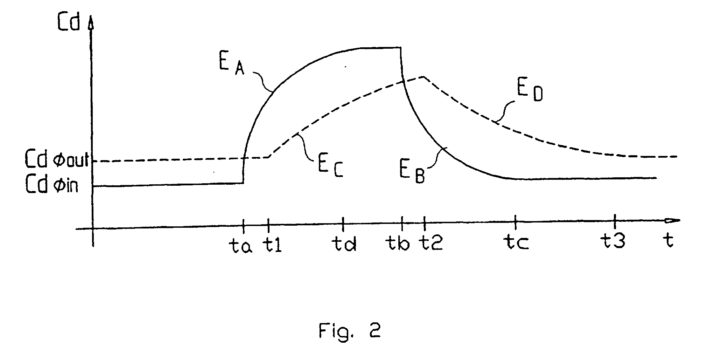

La figure 2 représente, de façon schématique, l'évolution en fonction du temps de la conductivité d'un liquide de dialyse en réponse à une augmentation de la concentration en sodium du liquide de dialyse pendant un temps déterminé tb - ta (échelon de conductivité). Le graphe en trait plein représente la conductivité en amont de l'hémodialyseur 1, telle que mesurée par la sonde de conductivité 23, et le graphe en trait interrompu représente la conductivité en aval de l'hémodialyseur 1, telle que mesurée par la sonde de conductivité 25.

Lorsque la conductivité du liquide de dialyse frais a repris sa valeur nominale Cd0in, l'unité de commande 30 calcule la superficie Sin de la zone de perturbation amont qui est délimitée par :

- une ligne de base constituée par la valeur nominale de la conductivité (Cd0in) du liquide de dialyse frais, et
- une courbe représentative de l'évolution par rapport au temps de la conductivité pendant la durée de la perturbation tc - ta, cette courbe étant établie à partir des valeurs Cdiin mesurées par la sonde 23 et échantillonnées et mises en mémoire par l'unité de commande 30.

Selon un mode de réalisation de l'invention, le calcul de la superficie Sin de la zone de perturbation amont consiste à effectuer la somme d'une pluralité de n surfaces élémentaires, chaque surface élémentaire étant égale au produit de l'intervalle de temps d'échantillonnage $\Delta t$ par chaque valeur échantillonnée Cdiin diminuée de la valeur de conductivité nominale Cd0in.

$$Sin = \Delta t \times \sum_{o}^{n} [Cdiin - Cd0in]$$

Selon un autre mode de réalisation, l'unité de commande 30 calcule la superficie Sin de la zone perturbation amont selon les étapes suivantes :

- détermination d'une équation de la courbe représentative de la variation, en fonction du temps, des valeurs Cdiin échantillonnées de la conductivité ;
- calcul de l'intégrale de cette équation entre le début et la fin de la variation de la conductivité ; et
- calcul de la superficie Sin de la zone perturbation amont, en soustrayant de l'intégrale calculée une superficie égale au produit de la durée de la perturbation tc - ta par la valeur nominale de la conductivité Cd0in.

La méthode pour déterminer l'équation de la courbe doit naturellement être choisie en fonction de la forme de la variation. A titre d'exemple, lorsque la variation de la conductivité résulte de l'augmentation (ou de la diminution) de la conductivité nominale Cd0in d'une quantité donnée pendant un laps de temps donné, suivi d'un retour à la conductivité nominale (échelon de conductivité), la courbe représentative de la variation, en fonction du temps, des valeurs échantillonnées Cdiin se compose de deux tronçons d'exponentielles comme cela apparaît clairement sur la figure 2. L'équation de chaque exponentielle peut être calculée avant même que soit atteint, pour la première exponentielle $E_A$, le point de jonction, intervenant à l'instant tb, de l'exponentielle $E_A$ à la deuxième exponentielle $E_B$, et, pour la l'exponentielle $E_B$ avant même le retour de la conductivité à sa valeur nominale Cd0in, qui intervient à l'instant tc.

Si on prend l'exemple de la première exponentielle $E_A$, on calcule de la façon suivante son équation entre ta et tb, $y_A = c + a \times e^{-bt}$, à partir de 3n valeurs Cbiin de conductivité consécutives échantillonnées entre l'instant ta et l'instant td.

- on effectue les trois sommes suivantes:

$$S1 = \sum_{1}^{n} Cdiin \qquad S2 = \sum_{n+1}^{2n} Cdiin \qquad S3 = \sum_{2n+1}^{3n} Cdiin$$

- on calcule la constante de temps b à partir de l'équation suivante:

$$1/b = n \times \Delta t/\ln \frac{(S1 - S2)}{(S2 - S3)}$$

- on calcule la valeur asymptotique c, à partir de l'équation suivante:

$$c = 1/n \times \frac{S1 \times S3 - S2^2}{S1 + S3 - 2 \times S2}$$

- connaissant c, on obtient le coefficient a immédiatement:

$$a = Cd0in - c$$

De la même manière, on calcule l'équation $y_B = c' + a' \times e^{-b't}$ de la deuxième exponentielle $E_B$ entre tb et tc.

La superficie Sin de la zone de perturbation amont délimitée entre les exponentielles $E_A$ et $E_B$ et la ligne de base constituée par la valeur nominale de la conductivité Cd0in peut alors être calculée par la formule :

$$Sin = \int_{ta}^{tb} (c + a \times e^{-bt}) \, dt + \int_{tb}^{tc} (c' + a' \times e^{-b't}) \, dt - (c + a) \times (tc - ta)$$

ou par la formule:

$$Sin = \int_{ta}^{tb} a \times (e^{-bt} - 1) \, dt + \int_{tb}^{tc} a' \times (e^{-b't} - 1) \, dt$$

Comme cela a été mentionné plus haut, selon une variante de l'invention, l'étape de détermination de la superficie Sin de la zone de perturbation amont est effectuée une fois pour toute pour une variation calibrée de la caractéristique du liquide de dialyse dont les paramètres (intensité, durée, profil) sont mis en mémoire dans l'unité de commande 30. La superficie Sin peut être calculée à partir de valeurs mesurées de la conductivité ou elle peut être calculée à partir de valeurs de consignes définissant la perturbation. Lorsque la superficie Sin de la zone de perturbation amont a été déterminée (soit préalablement à une séance de traitement particulière, soit au début d'une séance de traitement particulière), cette grandeur est mise en mémoire dans l'unité de commande 30 et, chaque fois que le procédé est mis en oeuvre ultérieurement, c'est cette grandeur qui est utilisée.

3- Détermination d'une grandeur significative de la variation de la caractéristique en aval de l'hémodialyseur 1, telle qu'elle apparaît déformée par les échanges ayant eu lieu dans l'hémodialyseur 1.

Conformément à l'invention, la grandeur choisie est la superficie Sout d'une zone appelée "zone de perturbation aval" qui est déterminée comme suit.

La conductivité du liquide de dialyse en amont de l'hémodialyseur 1 étant constante (Cd0in), la conductivité du liquide usé, telle que mesurée par la sonde 25, soit varie lentement de façon exponentielle, soit reste sensiblement constante (cas de la figure 2), selon qu'il existe une différence de conductivité appréciable entre le sang et le liquide de dialyse ou que cette différence est nulle ou quasi nulle. Dans la suite on se placera dans l'hypothèse où la conductivité du liquide usé reste sensiblement constante et égale à Cd0out. Cette valeur, mesurée par la sonde 25, est mise en mémoire.

Après qu'une variation de la conductivité du liquide de dialyse frais a été déclenchée comme expliqué plus haut, la quatrième sonde de conductivité 25 disposée sur la canalisation d'évacuation 10 mesure la conductivité en continu et l'unité de commande 30 échantillonne une pluralité de valeurs de la conductivité Cdjout du liquide de dialyse usé s'écartant de la conductivité initiale Cd0out.

Lorsque la conductivité du liquide de dialyse usé a repris sa valeur initiale Cd0out l'unité de commande 30 calcule la superficie Sout de la zone de perturbation aval qui est délimitée par :

- une ligne de base constituée par la valeur initiale Cd0out de la conductivité en aval de l'hémodialyseur 1, correspondant à la valeur nominale Cd0in de la conductivité en amont de l'hémodialyseur 1, et
- une courbe représentative de l'évolution par rapport au temps de la conductivité du liquide usé pendant la durée de la perturbation t3 - t1, cette courbe étant établie à partir des valeurs Cdjout mesurées par la sonde 25 et échantillonnées par l'unité de commande 30.

Le calcul de la superficie de la zone de perturbation Sout, peut être effectué par exemple par la méthode des surfaces élémentaires ou par la méthode de l'intégration telles qu'elles ont été décrites en détail à propos du calcul de la superficie Sin de la zone de perturbation amont.

Dans le cas où l'unité de commande 30 effectue le calcul selon la méthode des surfaces élémentaires, la superficie Sout de la zone de perturbation aval est obtenue par la formule:

$$Sout = \Delta t \times \sum_{o}^{m} [Cdjout - Cd0out]$$

Dans le cas où l'unité de commande 30 effectue le calcul selon la méthode de l'intégration, la superficie Sout de la zone de perturbation aval est obtenue par la formule:

$$\text{Sout} = \int_{t1}^{t2} q \times (e^{-rt} - 1)\, dt + \int_{t2}^{t3} q' \times (e^{-r't} - 1)\, dt$$

la courbe représentative de l'évolution de la conductivité du liquide de dialyse usé étant constituée de deux tronçons d'exponentielles $E_C$ et $E_D$ d'équations respectives $y_C = p + q \times e^{-rt}$ (entre t1 et t2) et $y_D = p' + q' \times e^{-r't}$ (entre t2 et t3).

Dans ce qui précède on s'est placé dans l'hypothèse où, pour une même valeur de la conductivité Cd0in en amont de l'hémodialyseur 1, la conductivité du liquide usé reste sensiblement constante. Si cette conductivité varie de façon appréciable, on utilise dans les calculs une ligne de base constituée soit par un tronçon d'exponentielle, soit par un tronçon de droite de pente positive ou négative constituant une approximation de cette exponentielle. L'équation de ce tronçon de droite peut être, par exemple, déterminée à partir d'une première valeur Cd1out mesurée à un instant t1, juste avant la perturbation, et d'une deuxième valeur Cd2out mesurée à un instant t3 auquel la conductivité en amont de l'hémodialyseur 1 a repris la valeur Cd0in et la perturbation a cessé de produire ses effets en aval de l'hémodialyseur1.

4- Calcul d'un paramètre significatif de l'efficacité du traitement administré.

Conformément à l'invention, le paramètre significatif de l'efficacité du traitement est calculé par l'unité de commande 30 à partir de la superficie Sin de la zone de perturbation amont et de la superficie Sout de la zone de perturbation aval.

Lorsque la caractéristique soumise à variation est la concentration dans le liquide de dialyse d'une substance présente dans le liquide de dialyse et dans le sang, telle que le sodium, l'unité de commande calcule la dialysance D de l'hémodialyseur pour cette substance au moyen de la formule:

$$D = (Qd + Quf) \times (1 - \text{Sout/Sin})$$

où Qd est le débit du liquide de traitement et Quf est le débit d'ultrafiltration.

La mise en oeuvre simultanée, à plusieurs reprises, du procédé selon l'invention et du procédé décrit dans le document EP 0 658 352 mentionné plus haut, qui permet en particulier de calculer la dialysance D, a permis de vérifier la grande fiabilité du procédé selon l'invention.

L'unité de commande 30 peut alors aussi calculer la concentration Cbin du sodium dans le sang du patient en amont de l'hémodialyseur au moyen de la formule:

$$\text{Cbin} = \frac{(\text{Cd0in} \times \text{Sout/Sin} + \text{Cd0out})}{(1 - \text{Sout/Sin})}$$

Lorsque la caractéristique soumise à variation est la concentration dans le liquide de dialyse d'une substance présente dans le sang et absente du liquide de dialyse, telle que l'urée, l'unité de commande calcule la clairance K de l'hémodialyseur pour cette substance au moyen de la formule:

$$K = (Qd + Quf) \times (1 - \text{Sout/Sin})$$

où Qd est le débit du liquide de traitement et Quf est le débit d'ultrafiltration.

L'unité de commande 30 peut alors aussi calculer la concentration Cbin de l'urée dans le sang du patient en amont de l'hémodialyseur au moyen de la formule:

$$\text{Cbin} = \frac{\text{Cd0out}}{(1 - \text{Sout/Sin})}$$

Exemple:

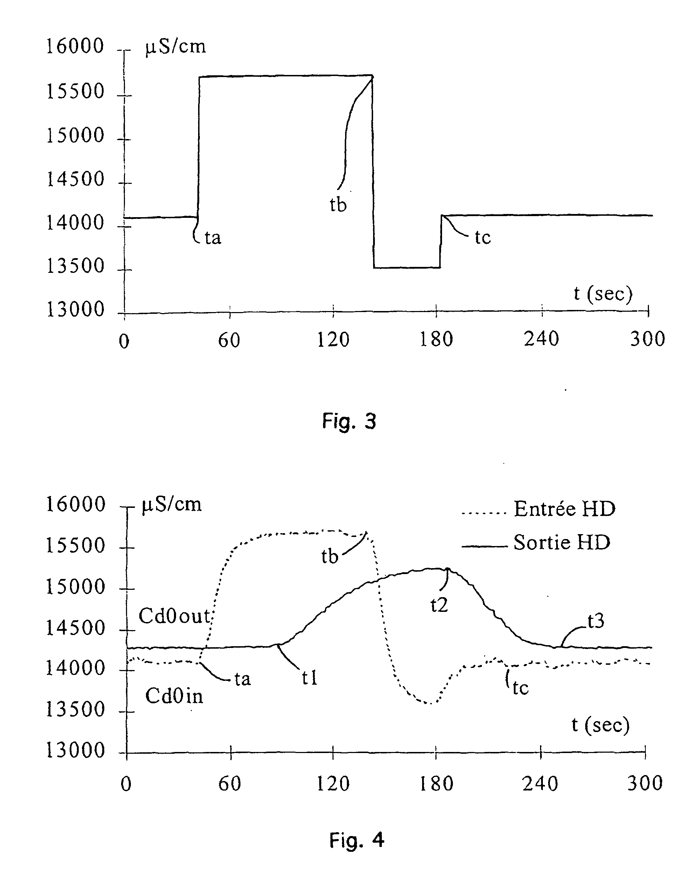

La figure 3 représente les valeurs de consigne qui ont été communiquées à l'unité de commande 30 pour régler la conductivité du liquide de dialyse à une valeur nominale de traitement et pour faire varier la conductivité, à intervalles de temps déterminés ou sur commande, pour provoquer une perturbation permettant de déterminer l'efficacité d'une séance de dialyse selon le procédé exposé ci-dessus.

La valeur de consigne nominale a été fixée à 14,1 mS/cm. La perturbation programmée comprend un échelon de conductivité de +1,6 mS/cm pendant 100 s suivi d'un contre-échelon de -0,6 mS/cm pendant 38 s à partir de la valeur de consigne nominale.

Un traitement de dialyse a été administré à un patient au moyen du système représenté sur la figure 1, équipé d'un dialyseur à membrane plane en AN69 du type CRYSTAL 4000 fabriqué par la société HOSPAL INDUSTRIE, Meyzieu, France. Les diverses pompes du système ont été pilotées de façon que le débit du sang Qb soit égal à 200 ml/min, le débit du liquide de dialyse Qd soit égal à 500 ml/min et le débit d'ultrafiltration Quf soit égal à 20 ml/min.

Au cours du traitement, on a demandé le déclenchement, par l'unité de commande 30, de la perturbation programmée de la conductivité.

Sur le graphe de la figure 4, sont représentées, en trait interrompu, l'évolution de la conductivité en amont du dialyseur 1, telle que mesurée par la sonde 23 toutes les 2 s ($\Delta t = 2$ s) et, en trait continu, l'évolution de la conductivité en aval du dialyseur, telle que mesurée par la sonde 25 toutes les 2 s. La valeur mesurée de la conductivité Cd0in en amont du dialyseur, avant et après la perturbation, est égale à 14,113 mS/cm et la valeur mesurée de la conductivité Cd0out en aval du dialyseur, avant et après la perturbation est égale à 14,28 mS/cm. En d'autres termes, en fonctionnement normal, un transfert de substances ionisées a lieu dans le dialyseur du sang vers le liquide de dialyse. La durée tc - ta de la perturbation en amont de l'échangeur est égale à 185 s et la durée de la perturbation t3 - t1 en aval de l'échangeur est égale à 168 s. La superficie (Sin) de la zone de perturbartion amont, calculée par la méthode des aires, est égale à :

$$\text{Sin} = \Delta t \times \Sigma \, [\text{Cdiin - Cd0in}] =$$

$$2 \times \Sigma \, [\text{Cdiin 14,113}] = 174,45 \text{ mS/cm} \times s$$

La superficie (Sout) de la zone de perturbation en aval, calculée par la méthode des aires est égale à :

$$\text{Sout} = \Delta t \times \Sigma \, [\text{Cdjout - Cd0out}] =$$

$$2 \times \Sigma \, [\text{Cdjout - 14,281}] = 114,984 \text{ mS/cm} \times s$$

La dialysance D du système est égale à :

$$D = (Qd + Quf) \times (1 - \text{Sout/Sin}) = 177 \text{ ml/min}$$

On note que le patient n'a été exposé à un liquide de dialyse ayant une conductivité différente de la conductivité nominale que pendant un temps très court (168 s, qui sont à rapporter aux quatre heures que dure une séance de dialyse classique). On note aussi que le temps nécessaire pour déterminer la dialysance D du système de dialyse, qui est approximativement égal au temps t3 - ta nécessaire pour effectuer les mesures est lui aussi très court, soit 216 s, de sorte que plusieurs déterminations de la dialysance D peuvent être effectuées par heure si jugé nécessaire.

L'invention n'est pas limitée aux exemples de réalisation qui viennent d'être décrits et elle est susceptible de variantes.

**Revendications**

1. Procédé de détermination d'un paramètre (D, K, Kt/v, Cbin) significatif de l'efficacité d'un traitement extracorporel du sang consistant à faire circuler de part et d'autre de la membrane semi-perméable (4) d'un échangeur (1) à membrane le sang d'un patient et un liquide de traitement, **caractérisé en ce qu'**il comporte les étapes de :

   - faire circuler dans l'échangeur (1) un liquide de traitement ayant une caractéristique (Cd) ayant une valeur nominale (Cd0in) sensiblement constante en amont de l'échangeur (1);
   - faire varier la valeur de la caractéristique (Cd) en amont de l'échangeur (1) pendant une durée tc - ta, à la fin de laquelle la caractéristique (Cd) est rétablie à sa valeur nominale (Cd0in) en amont de l'échangeur (1);

- mesurer et mettre en mémoire une pluralité de valeurs prises par la caractéristique (Cd) du liquide de traitement en aval de l'échangeur (1) en réponse à la variation de la valeur de cette caractéristique (Cd) provoquée en amont de l'échangeur (1 );

**caractérisé en ce qu'**il comporte les étapes de:

- déterminer la superficie (Sout) d'une zone de perturbation aval délimitée par:

- une ligne de base passant par deux valeurs prises par la caractéristique (Cd) à deux instants encadrant la perturbation et auxquels la caractéristique (Cd) n'est pas, respectivement plus, influencée par la perturbation, et
- une courbe représentative de l'évolution par rapport au temps de la caractéristique (Cd), établie à partir des valeurs de la caractéristique (Cd) mesurées en aval de l'échangeur (1) entre deux instants t1 et t3 encadrant la perturbation et auxquels la caractéristique (Cd) n'est pas, respectivement plus, influencée par la perturbation ; et

- calculer le paramètre (D, K, Kt/v, Cbin) significatif de l'efficacité d'un traitement à partir de la superficie (Sout) de la zone de perturbation aval et de la superficie (Sin) d'une zone de perturbation amont délimitée par:

- une ligne de base constituée par la valeur nominale (Cd0in) de la caractéristique (Cd), et
- une courbe représentative de l'évolution par rapport au temps, pendant la durée tc - ta, de la caractéristique (Cd) en amont de l'échangeur (1).

2. Procédé selon la revendication 1, **caractérisé en ce que** l'étape de faire varier la valeur de la caractéristique (Cd) du liquide de traitement comprend l'étape de régler, pendant un intervalle de temps déterminé, la caractéristique à une valeur de consigne déterminée supérieure ou inférieure à la valeur nominale (Cd0in).

3. Procédé selon la revendication 1, **caractérisé en ce que** l'étape de faire varier la valeur de la caractéristique (Cd) du liquide de traitement en amont de l'échangeur (1) comprend les étapes de :

- régler, pendant un premier intervalle de temps déterminé, la caractéristique à une première valeur de consigne déterminée supérieure (respectivement inférieure) à la valeur nominale (Cd0in), et
- régler, pendant un deuxième intervalle de temps, la caractéristique à une deuxième valeur de consigne inférieure (respectivement supérieure) à la valeur nominale (Cd0in).

4. Procédé selon la revendication 3, **caractérisé en ce qu'**il comprend en outre l'étape de choisir la durée du deuxième intervalle de temps ainsi que la valeur absolue de la deuxième valeur de consigne de façon que la caractéristique (Cd) reprenne sa valeur initiale (Cd0out) en aval de l'échangeur (1) en un temps minimal.

5. Procédé selon une des revendications 1 à 4, **caractérisé en ce qu'**il comprend en outre, préalablement à l'étape de calculer le paramètre significatif de l'efficacité du traitement extracorporel, l'étape de déterminer la superficie (Sin) de la zone de perturbation amont.

6. Procédé selon une des revendications 1 à 4, **caractérisé en ce qu'**il comprend en outre, préalablement à l'étape de faire varier la valeur de la caractéristique (Cd), les étapes de:

- donner aux paramètres de la variation une valeur définie, de façon que la superficie de la zone de perturbation amont Sin soit constante quand les paramètres prédéterminés de la variation sont respectés;
- déterminer et mettre en mémoire la superficie de la zone de perturbation amont Sin pour une variation ayant les paramètres fixés.

7. Procédé selon une des revendications 5 ou 6, **caractérisé en ce qu'**il comprend en outre, préalablement à l'étape de déterminer la superficie de la zone de perturbation amont Sin, l'étape de mesurer une pluralité de valeurs prises par la caractéristique (Cd) du liquide de traitement en amont de l'échangeur (1) pendant la durée tc - ta.

8. Procédé selon une des revendications 1 à 7, **caractérisé en ce que** l'étape de déterminer la superficie Sin (Sout) de la zone de perturbation amont (respectivement aval) comprend les étapes de :

- déterminer une équation de la courbe représentative de la variation, en fonction du temps, des valeurs mesurées de la caractéristique (Cd) en amont (respectivement en aval) de l'échangeur (1); et
- calculer l'intégrale de cette équation entre le début et la fin de la variation de la valeur de la caractéristique (Cd); et
- retrancher à l'intégrale de l'équation une superficie égale au produit de la durée de la perturbation amont (respectivement aval) par la valeur nominale Cd0in (respectivement la valeur initiale Cd0out) de la caractéristique en amont (respectivement en aval) de l'échangeur (1).

9. Procédé selon une des revendications 1 à 7, **caractérisé en ce que** l'étape de déterminer la superficie (Sin, Sout) d'une zone de perturbation comprend les étapes de :

- mesurer la valeur de la caractéristique Cd à intervalles de temps $\Delta t$ pendant toute la durée de la perturbation;
- calculer une série de superficies élémentaires en multipliant, par l'intervalle de temps $\Delta t$, chaque valeur mesurée diminuée de la valeur nominale (Cd0in) (respectivement de la valeur initiale Cd0out) de la caractéristique Cd;
- faire la somme des superficies élémentaires.

10. Procédé selon une des revendications 1 à 9, **caractérisé en ce que** la caractéristique soumise à variation est la concentration dans le liquide de traitement d'une substance présente dans le liquide de traitement et dans le sang, et **en ce que** la dialysance D de l'échangeur (1 ) pour cette substance est calculée au moyen de la formule:

$$D = (Qd + Quf) \times (1 - Sout/Sin)$$

où Qd est le débit du liquide de traitement et Quf est le débit d'ultrafiltration.

11. Procédé selon une des revendications 1 à 10, **caractérisé en ce que** la caractéristique soumise à variation est la concentration dans le liquide de traitement d'une substance présente dans le liquide de traitement et dans le sang, et **en ce que** la concentration Cbin de la substance dans le sang du patient en amont de l'échangeur (1) est calculée au moyen de la formule :

$$Cbin = \frac{(Cd0in \times Sout/Sin + Cd0out)}{(1 - Sout/Sin)}$$

12. Procédé selon une des revendications 1 à 9, **caractérisé en ce que** la caractéristique soumise à variation est la concentration dans le liquide de traitement d'une substance présente dans le sang et absente du liquide de traitement, et **en ce que** la clairance K de l'échangeur (1) pour cette substance est calculée au moyen de la formule:

$$K = (Qd + Quf) \times (1 - Sout/Sin)$$

où Qd est le débit du liquide de traitement et Quf est le débit d'ultrafiltration.

13. Procédé selon une des revendications 1 à 9, **caractérisé en ce que** la caractéristique soumise à variation est la concentration dans le liquide de traitement d'une substance présente dans le sang et absente du liquide de traitement, et **en ce que** la concentration Cbin de la substance dans le sang du patient en amont de l'échangeur (1) est calculée au moyen de la formule :

$$Cbin = \frac{Cd0out}{(1 - Sout/Sin)}$$

14. Procédé selon une des revendications 10 à 13, **caractérisé en ce que** l'étape de faire varier la valeur de la caractéristique (Cd) consiste à injecter dans le liquide de traitement en amont de l'échangeur (1) un volume d'un liquide contenant la substance.

15. Procédé selon la revendication 14, **caractérisé en ce qu'**une quantité déterminée de la substance est injecté dans le liquide de traitement, de sorte que la superficie de la zone de perturbation amont Sin peut être prédéterminée.

**16.** Procédé selon la revendication 10 ou 11, **caractérisé en ce que** l'étape de faire varier la concentration d'une substance dans le liquide de traitement consiste à modifier le débit d'une pompe (19) pour solution concentrée d'un générateur (11) de liquide de traitement ou une solution concentrée est mélangée à de l'eau ou à une solution diluée pour préparer le liquide de traitement.

**17.** Procédé selon la revendication 10 ou 11, **caractérisé en ce que** la substance est le sodium.

**18.** Procédé selon la revendication 12 ou 13, **caractérisé en ce que** la substance est l'urée.

**Claims**

**1.** Method of determining a parameter (D, K, Kt/v, Cbin) indicative of the effectiveness of an extracorporeal treatment of blood, consisting in making a patient's blood and a treatment liquid flow one on each side of the semipermeable membrane (4) of a membrane exchanger (1), **characterized in that** it includes the steps of:

- making a treatment liquid flow through the exchanger, a treatment liquid having a characteristic (Cd) which has an approximately constant nominal value (Cd0in) upstream of the exchanger (1);
- varying the value of the characteristic (Cd) upstream of the exchanger (1) for a time tc - ta, at the end of which the characteristic (Cd) is returned to its nominal value (Cd0in) upstream of the exchanger (1) ;
- measuring and storing in memory a plurality of values adopted by the characteristic (Cd) of the treatment liquid downstream of the exchanger (1) in response to the variation in the value of this characteristic (Cd) caused upstream of the exchanger (1) ;

**characterized in that** it includes the steps of:

- determining the area (Sout) of a downstream perturbation region bounded by:

  • a baseline passing through two values given by the characteristic (Cd) at two times flanking the perturbation, at which times the characteristic (Cd) is not, or respectively is no longer, influenced by the perturbation; and
  • a curve representing the variation with respect to time of the characteristic (Cd), established from the values of the characteristic (Cd) which are measured downstream of the exchanger (1) between two times t1 and t3 flanking the perturbation, at which times the characteristic (Cd) is not, or respectively is no longer, influenced by the perturbation; and

- calculating the parameter (D, K, Kt/v, Cbin) indicative of the effectiveness of a treatment from the area (Sout) of the downstream perturbation region and from the area (Sin) of an upstream perturbation region bounded by:

  • a baseline consisting of the nominal value (Cd0in) of the characteristic (Cd), and
  • a curve representing the variation with respect to time, over the period tc - ta, of the characteristic (Cd) upstream of the exchanger (1).

**2.** Method according to Claim 1, **characterized in that** the step of varying the value of the characteristic (Cd) of the treatment liquid comprises the step of setting the characteristic, over a defined time interval, to a defined set value above or below the nominal value (Cd0in).

**3.** Method according to Claim 1, **characterized in that** the step of varying the value of the characteristic (Cd) of the treatment liquid upstream of the exchanger (1) comprises the steps of:

- setting the characteristic, over a first defined time interval, to a first defined set value above (respectively below) the nominal value (Cd0in), and
- setting the characteristic, over a second time interval, to a second set value below (respectively above) the nominal value (Cd0in).

**4.** Method according to Claim 3, **characterized in that** it furthermore comprises the step of choosing the duration of the second time interval as well as the absolute value of the second set value so that the characteristic (Cd) resumes its initial value (Cd0out) downstream of the exchanger (1) in a minimum amount of time.

5. Method according to one of Claims 1 to 4, **characterized in that** it furthermore comprises, prior to the step of calculating the parameter indicative of the effectiveness of the extracorporeal treatment, the step of determining the area (Sin) of the upstream perturbation region.

6. Method according to one of Claims 1 to 4, **characterized in that** it furthermore comprises, prior to the step of varying the value of the characteristic (Cd), the steps of:

   - giving the parameters of the variation a defined value, so that the area of the upstream perturbation region Sin is constant when the predetermined parameters of the variation are complied with;
   - determining and storing in memory the area of the upstream perturbation region Sin for a variation having the fixed parameters.

7. Method according to either of Claims 5 and 6, **characterized in that** it furthermore comprises, prior to the step of determining the area of the upstream perturbation region Sin, the step of measuring a plurality of values adopted by the characteristic (Cd) of the treatment liquid upstream of the exchanger (1) during the time period tc - ta.

8. Method according to one of Claims 1 to 7, **characterized in that** the step of determining the area Sin (Sout) of the upstream (respectively downstream) perturbation region comprises the steps of:

   - determining an equation for the curve representing the variation, as a function of time, in the measured values of the characteristic (Cd) upstream (respectively downstream) of the exchanger (1); and
   - calculating the integral of this equation between the start and end of the variation in the value of the characteristic (Cd); and
   - subtracting from the integral of the equation an area equal to the product of the duration of the upstream (respectively downstream) perturbation and the nominal value Cd0in (respectively the initial value Cd0out) of the characteristic upstream (respectively downstream) of the exchanger (1).

9. Method according to one of Claims 1 to 7, **characterized in that** the step of determining the area (Sin, Sout) of a perturbation region comprises the steps of:

   - measuring the value of the characteristic Cd at time intervals $\Delta t$ throughout the duration of the perturbation;
   - calculating a series of elementary areas by multiplying each measured value, decreased by the nominal value (Cd0in) (respectively by the initial value Cd0out) of the characteristic Cd, by the time interval $\Delta t$; and
   - summing the elementary areas.

10. Method according to one of Claims 1 to 9, **characterized in that** the characteristic subjected to variation is the concentration in the treatment liquid of a substance present in both the treatment liquid and the blood, and **in that** the dialysance D of the exchanger (1) for this substance is calculated by means of the formula:

$$D = (Qd + Quf) \times (1- Sout/Sin)$$

where Qd is the flow rate of the treatment liquid and Quf is the ultrafiltration flow rate.

11. Method according to one of Claims 1 to 10, **characterized in that** the characteristic subjected to variation is the concentration in the treatment liquid of a substance present in the treatment liquid and in the blood and **in that** the concentration Cbin of the the substance in the patient's blood upstream of the exchanger (1) is calculated by means of the formula:

$$Cbin = \frac{Cd0out}{(1-Sout / Sin)}$$

12. Method according to one of Claims 1 to 9, **characterized in that** the characteristic subjected to variation is the concentration in the treatment liquid of a substance present in the blood and absent from the treatment liquid, and **in that** the clearance K of the exchanger (1) for this substance is calculated by means of the formula:

$$K = (Qd + Quf) \times (1- Sout / Sin)$$

where Qd is the flow rate of the treatment liquid and

Quf if the ultrafiltration flow rate.

**13.** Method according to one of Claims 1 to 9, **characterized in that** the characteristic subjected to variation is the concentration in the treatment liquid of a substance present in the blood and absent from the treatment liquid and **in that** the concentration Cbin of the substance in the patient's blood upstream of the exchanger (1) is calculated by means of the formula:

$$Cbin = \frac{Cd0out}{(1\text{-}Sout\,/\,Sin)}$$

**14.** Method according to one of Claims 10 to 13, **characterized in that** the step of varying the value of the characteristic (Cd) consists in injecting a volume of a liquid containing the substance into the treatment liquid upstream of the exchanger (1)

**15.** Method according to Claim 14, **characterized in that** a defined quantity of the substance is injected into the treatment liquid so that the area of the upstream perturbation region Sin can be predetermined.

**16.** Method according to Claim 10 or 11, **characterized in that** the step of varying the concentration of a substance in the treatment liquid consists in modifying the discharge rate of a pump (19) for a concentrated solution of a treatment-liquid generator (11) in which a concentrated solution is mixed with water or with a dilute solution, in order to prepare the treatment liquid.

**17.** Method according to Claim 10 or 11, **characterized in that** the substance is sodium.

**18.** Method according to Claim 12 or 13, **characterized in that** the substance is urea.


**Patentansprüche**

**1.** Verfahren zur Bestimmung eines signifikanten Parameters (D, K, Kt/V, Cbin) für die Wirksamkeit einer extrakorporalen Behandlung des Blutes, das darin besteht, beiderseits der semipermeablen Membran (4) eines Membranaustauschers (1) das Blut eines Patienten und eine Behandlungsflüssigkeit zirkulieren zu lassen, **dadurch gekennzeichnet, dass** es die folgenden Schritte umfasst:

- Zirkulation einer Behandlungsflüssigkeit in dem Austauscher (1), die ein Merkmal (Cd) aufweist, das einen im Wesentlichen konstanten Nominalwert (CdOin) stromaufwärts zum Austauscher (1) aufweist;

- Veränderung des Werts des Merkmals (Cd) stromaufwärts zum Austauscher (1) während einer Dauer tc - ta, nach der das Merkmal (Cd) wieder auf seinen Nominalwert (CdOin) stromaufwärts zum Austauscher (1) gebracht wird;

- Messung und Speicherung einer Vielzahl von Werten, die von dem Merkmal (Cd) der Behandlungsflüssigkeit stromabwärts zum Austauscher (1) als Antwort auf die Veränderung des Wertes dieses Merkmals (Cd), die stromaufwärts zum Austauscher (1) herbeigeführt wurde, genommen wurden;

**dadurch gekennzeichnet, dass** es die folgenden Schritte umfasst:

- Bestimmung der Oberfläche (Sout) einer stromabwärtigen Störzone, die begrenzt ist durch:

  • eine Basislinie, die durch zwei Werte geht, die von dem Merkmal (Cd) zu zwei Zeitpunkten, die die Störung umgeben, genommen wurden, zu denen das Merkmal (Cd) nicht bzw. nicht mehr von der Störung beeinflusst ist, und

  • eine für die Entwicklung des Merkmals (Cd) im Verhältnis zur Zeit repräsentative Kurve, die auf Basis der Werte des Merkmals (Cd) erstellt wurde, die stromabwärts zum Austauscher (1) zwischen zwei Zeitpunkten t1 und t3, die die Störung umgeben, genommen wurden, zu denen das Merkmal (Cd) nicht bzw. nicht mehr von der Störung beeinflusst ist; und

- Berechnung des Parameters (D, K, Kt/V, Cbin), der für die Wirksamkeit einer Behandlung signifikant ist, auf Basis der Oberfläche (Sout) einer stromabwärtigen Störzone und der Oberfläche (Sin) einer stromaufwärtigen Störzone, die begrenzt ist durch:

  • eine Basislinie, die von dem Nominalwert (CdOin) des Merkmals (Cd) gebildet ist, und

  • eine Kurve, die für die Entwicklung des Merkmals (Cd) im Verhältnis zur Zeit während der Dauer tc - ta stromaufwärts zum Austauscher (1) repräsentativ ist.

2. Verfahren nach Anspruch 1, **dadurch gekennzeichnet, dass** der Schritt der Veränderung des Wertes des Merkmals (Cd) der Behandlungsflüssigkeit den Schritt der Einstellung des Merkmals auf einen bestimmten Sollwert, der größer oder kleiner als der Nominalwert (CdOin) ist, während eines bestimmten Zeitintervalls umfasst.

3. Verfahren nach Anspruch 1, **dadurch gekennzeichnet, dass** der Schritt der Veränderung des Wertes des Merkmals (Cd) der Behandlungsflüssigkeit stromaufwärts zum Austauscher (1) die folgenden Schritte umfasst:

   - während eines ersten bestimmten Zeitintervalls Einstellung des Merkmals auf einen ersten bestimmten Sollwert, der größer (bzw. kleiner) als der Nominalwert (CdOin) ist, und

   - während eines zweiten Zeitintervalls Einstellung des Merkmals auf einen zweiten Sollwert, der kleiner (bzw. größer) als der Nominalwert (CdOin) ist.

4. Verfahren nach Anspruch 3, **dadurch gekennzeichnet, dass** es ferner den Schritt der Wahl der Dauer des zweiten Zeitintervalls sowie des Absolutwertes des zweiten Sollwerts umfasst, so dass das Merkmal (Cd) wieder seinen ursprünglichen Wert (CdOout) stromabwärts zum Austauscher (1) innerhalb einer minimalen Zeit annimmt.

5. Verfahren nach einem der Ansprüche 1 bis 4, **dadurch gekennzeichnet, dass** es ferner vor dem Schritt der Berechnung des signifikanten Parameters für die Wirksamkeit der extrakorporalen Behandlung den Schritt der Bestimmung der Oberfläche (Sin) der stromaufwärtigen Störzone umfasst.

6. Verfahren nach einem der Ansprüche 1 bis 4, **dadurch gekennzeichnet, dass** es ferner vor dem Schritt der Veränderung des Wertes des Merkmals (Cd) die folgenden Schritte umfasst:

   - Festlegung eines definierten Wertes für die Parameter der Veränderung, so dass die Oberfläche der stromaufwärtigen Störzone Sin konstant ist, wenn die vorbestimmten Parameter der Veränderung eingehalten werden;

   - Bestimmung und Speicherung der Oberfläche der stromaufwärtigen Störzone Sin bei einer Veränderung mit den festgelegten Parametern.

7. Verfahren nach Anspruch 5 oder 6, **dadurch gekennzeichnet, dass** es ferner vor dem Schritt der Bestimmung der Oberfläche der stromaufwärtigen Störzone Sin den Schritt der Messung einer Vielzahl von Parametern umfasst, die von dem Merkmal (Cd) der Behandlungsflüssigkeit stromaufwärts zum Austauscher (1) während der Dauer tc - ta genommen wurden.

8. Verfahren nach einem der Ansprüche 1 bis 7, **dadurch gekennzeichnet, dass** der Schritt der Bestimmung der Oberfläche Sin (Sout) der stromaufwärtigen (bzw. stromabwärtigen) Störzone die folgenden Schritte umfasst:

   - Bestimmung einer Gleichung der Kurve, die für die zeitabhängige Veränderung der gemessenen Werte des Merkmals (Cd) stromaufwärts (bzw. stromabwärts) zum Austauscher (1) repräsentativ ist; und

   - Berechnung des Integrals dieser Gleichung zwischen dem Beginn und dem Ende der Veränderung des Wertes des Merkmals (Cd); und

   - beim Integral der Gleichung Abziehen einer Oberfläche gleich dem Produkt der Dauer der stromaufwärtigen (bzw. stromabwärtigen) Störung mal den Nominalwert CdOin (bzw. den ursprünglichen Wert CdOout) des Merkmals stromaufwärts (bzw. stromabwärts) zum Austauscher (1).

9. Verfahren nach einem der Ansprüche 1 bis 7, **dadurch gekennzeichnet, dass** der Schritt der Bestimmung der Oberfläche (Sin, Sout) einer Störzone die folgenden Schritte umfasst:

- Messung des Wertes des Merkmals Cd in Zeitintervallen $\Delta t$ während der gesamten Dauer der Störung;

- Berechnung einer Reihe von elementaren Oberflächen, indem jeder gemessene Wert, vermindert um den Nominalwert (CdOin) (bzw. den ursprünglichen Wert CdOout) des Merkmals Cd mit dem Zeitintervall $\Delta t$ multipliziert wird;

- Summieren der elementaren Oberflächen.

10. Verfahren nach einem der Ansprüche 1 bis 9, **dadurch gekennzeichnet, dass** das der Veränderung unterzogene Merkmal die Konzentration einer in der Behandlungsflüssigkeit und im Blut vorhandenen Substanz in der Flüssigkeit ist, und dass die Dialysierfähigkeit D des Austauschers (1) für diese Substanz mit Hilfe folgender Formel berechnet wird:

$$D = (Qd + Quf) \times (1 - Sout \,/\, Sin) \,,$$

wobei Qd die Durchflussmenge der Behandlungsflüssigkeit und Quf die Ultrafiltrationsmenge ist.

11. Verfahren nach einem der Ansprüche 1 bis 10, **dadurch gekennzeichnet, dass** das der Veränderung unterzogene Merkmal die Konzentration einer in der Behandlungsflüssigkeit und im Blut vorhandenen Substanz in der Behandlungsflüssigkeit ist, und dass die Konzentration Cbin der Substanz im Blut des Patienten stromaufwärts zum Austauscher (1) mit Hilfe folgender Formel berechnet wird:

$$Cbin = \frac{(CdOin \times Sout \,/\, Sin + CdOout)}{(1 - Sout \,/\, Sin)} \,.$$

12. Verfahren nach einem der Ansprüche 1 bis 9, **dadurch gekennzeichnet, dass** das der Veränderung unterzogene Merkmal die Konzentration einer im Blut vorhandenen und in der Behandlungsflüssigkeit fehlenden Substanz in der Behandlungsflüssigkeit ist, und dass die Ausscheidungsfähigkeit K des Austauschers (1) für diese Substanz mit Hilfe der folgenden Formel berechnet wird:

$$K = (Qd + Quf) \times (1 - Sout \,/\, Sin) \,,$$

wobei Qd die Durchflussmenge der Behandlungsflüssigkeit und Quf die Ultrafiltrationsmenge ist.

13. Verfahren nach einem der Ansprüche 1 bis 9, **dadurch gekennzeichnet, dass** das der Veränderung unterzogene Merkmal die Konzentration einer im Blut vorhandenen und in der Behandlungsflüssigkeit fehlenden Substanz in der Behandlungsflüssigkeit ist, und dass die Konzentration Cbin der Substanz im Blut des Patienten stromaufwärts zum Austauscher (1) mit Hilfe der folgenden Formel berechnet wird:

$$Cbin = \frac{CdOout}{(1 - Sout \,/\, Sin)} \,.$$

14. Verfahren nach einem der Ansprüche 10 bis 13, **dadurch gekennzeichnet, dass** der Schritt der Veränderung des Wertes des Merkmals (Cd) darin besteht, in die Behandlungsflüssigkeit stromaufwärts zum Austauscher (1) ein die Substanz enthaltendes Flüssigkeitsvolumen einzuspritzen.

15. Verfahren nach Anspruch 14, **dadurch gekennzeichnet, dass** eine bestimmte Menge der Substanz in die Behandlungsflüssigkeit eingespritzt wird, so dass die Oberfläche der stromaufwärtigen Störzone Sin vorbestimmt werden kann.

16. Verfahren nach Anspruch 10 oder 11, **dadurch gekennzeichnet, dass** der Schritt der Veränderung der Konzentration einer Substanz in der Behandlungsflüssigkeit darin besteht, die Durchflussmenge einer Pumpe (19) für eine konzentrierte Lösung eines Erzeugers (11) von Behandlungsflüssigkeit zu verändern oder eine konzentrierte Lösung mit Wasser oder einer verdünnten Lösung zu mischen, um die Behandlungsflüssigkeit herzustellen.

**17.** Verfahren nach Anspruch 10 oder 11, **dadurch gekennzeichnet, dass** die Substanz Natrium ist.

**18.** Verfahren nach Anspruch 12 oder 13, **dadurch gekennzeichnet, dass** die Substanz Harnstoff ist.

Fig. 1

UNITE DE COMMANDE

EP 0 920 877 B1

19

Fig. 2

**Fig. 3**

**Fig. 4**